# EUROPEAN PATENT SPECIFICATION

(11) **EP 3 509 552 B1**
(45) Date of publication and mention of the grant of the patent: **22.06.2022**
(21) Application number: 17848234.5
(22) Date of filing: 05.09.2017
(51) Int. Cl.: A61F 13/49, A61F 13/15, A61F 13/551

(54) **ABSORBENT ARTICLE WITH ADJUSTABLE CLOSURE**
SAUGFÄHIGER ARTIKEL MIT ANPASSBAREM VERSCHLUSS
ARTICLE ABSORBANT À FERMETURE AJUSTABLE

(30) Priority: 06.09.2016 US 201662383966 P
(43) Date of publication of application: 17.07.2019
(73) Proprietor: Associated Hygienic Products LLC, Greenville, North Carolina 27834 (US)
(72) Inventor: SCHROER, JR., Charles F., Greenville, North Carolina 27834 (US); KAISER, Thomas A., Greenville, North Carolina 27834 (US)
(74) Representative: Lohr, Jöstingmeier & Partner
(86) International application number: PCT/IB2017/055345
(87) International publication number: WO 2018/047065

(56) References cited:
- EP-A1- 1 702 598
- WO-A1-97/36566
- US-A- 5 295 986
- US-A- 5 295 986
- US-A1- 2001 041 879
- US-A1- 2004 082 931
- US-A1- 2006 129 121
- US-A1- 2015 032 077
- US-A1- 2016 228 302
- US-A1- 2016 228 302

## Description

### CROSS-REFERENCE TO RELATED APPLICATIONS

### FIELD OF INVENTION

The present invention relates generally to disposable absorbent products such as infant diapers, adult incontinence briefs, and pull-up underwear; and more particularly, but not by way of limitation, to such products with adjustable closures.

### BACKGROUND

Examples of disposable absorbent articles that are wearable by a user include baby diapers, training pants, and adult incontinence briefs and underwear, all of which may be made in disposable forms. "Disposable" refers to articles which are designed to be discarded after a limited use rather than being laundered or otherwise restored for reuse. Disposable absorbent products have met with widespread acceptance in the marketplace for a variety of applications, including infant and adult incontinence care, in view of the manner in which such products can provide effective and convenient liquid absorption and retention while maintaining the comfort of the wearer. Such disposable absorbent articles often include a topsheet that is configured to be closest to the wearer during use, a liquid-impermeable backsheet or outer cover, and an absorbent core between the topsheet and the backsheet. In some instances, such disposable absorbent articles also include an acquisition-distribution layer (ADL) disposed between the topsheet and the absorbent core.

U.S. Patent No. 9,398,986 discloses certain prior art examples of training pants, and U.S. Patents No. 6,976,978 and No. 4,940,464 disclose certain prior art examples of disposable incontinence garments or training pants.

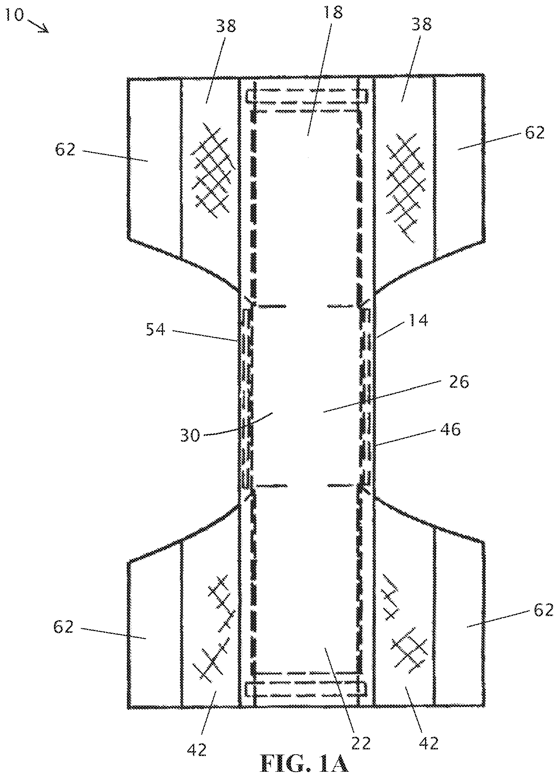

One example of such a disposable absorbent article is shown in FIGS. 1A-1B, which depict a lower plan view and a perspective view, respectively, of a training pant 10. Training pant 10 includes a chassis 14 having a front waist portion 18, an opposing rear waist portion 22, and a crotch portion 26 extending longitudinally between front and rear waist portions 18, 22. Chassis 14 further includes an outer surface 30 configured to face away from a wearer during use of the diaper, and an opposing body facing surface 34 configured to face a wearer during use of the diaper.

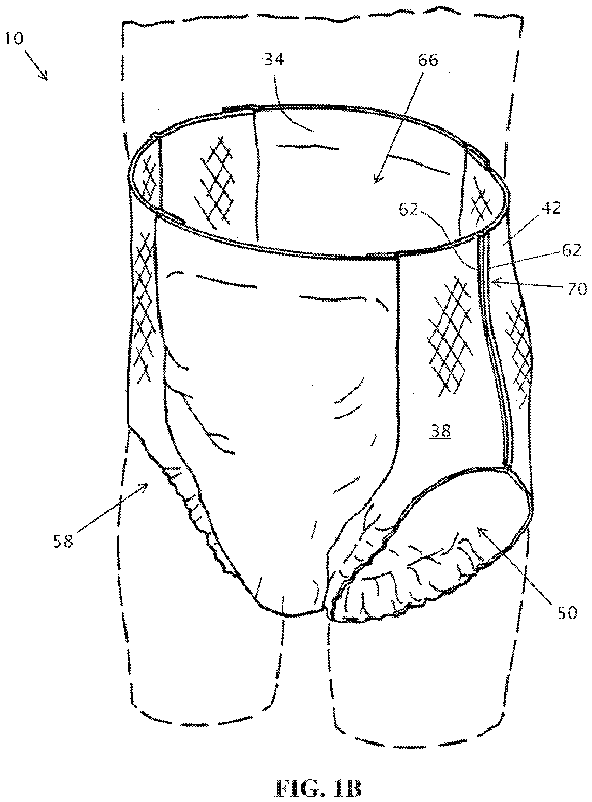

As shown in FIGS. 1A-1B, training pant 10 further includes a pair of front elastic side panels 38 and a pair of rear elastic side panels 42 configured to couple rear waist portion 22 to front waist portion 18 in a well-known configuration in which a left side 46 of the chassis defines a first leg opening 50 for a wearer's left leg, and in which a right side 54 of the chassis defines a second leg opening 58 for the wearer's right leg. In the depicted configuration, each of side panels 38, 42 includes a connection portion 62 configured to be coupled to a connection portion 62 of another of side panels 38, 42. Specifically, connection portion 62 of the left one of front side panels 38 is configure to be coupled to connection portion 62 of the left one of rear side panels 42, and connection portion 62 of the right one of front side panels 38 is configure to be coupled to connection portion 62 of the right one of rear side panels 42, such that the waist portions 18, 22 and side panels, 38, 42 cooperate to define a waist opening 66 as shown in FIG. 1B. Connection portions 62 of the respective side panels can be permanently coupled together to define a tear-able side seam 70, such as, for example, via adhesive, ultrasonic, or thermal bonds. Such tear-able side seams generally cannot be refastened, and thereby render an article unusable once opened. Alternatively, connection portions 62 of the respective side panels can be removably coupled to define a refastenable or adjustable side seam, such as, for example, via hook-and-loop fasteners. Hook and loop fasteners are mechanical fasteners that include hooks, such as in a hook fastener portion, that are configured to engage loops in a loop fastener portion or in fibers of a sheet of fabric; for example, a nonwoven or woven fabric with fibers that define open or loop-like regions into which the hooks can extend and engage. Examples of such hook and loop fasteners may be referred to as VELCRO.

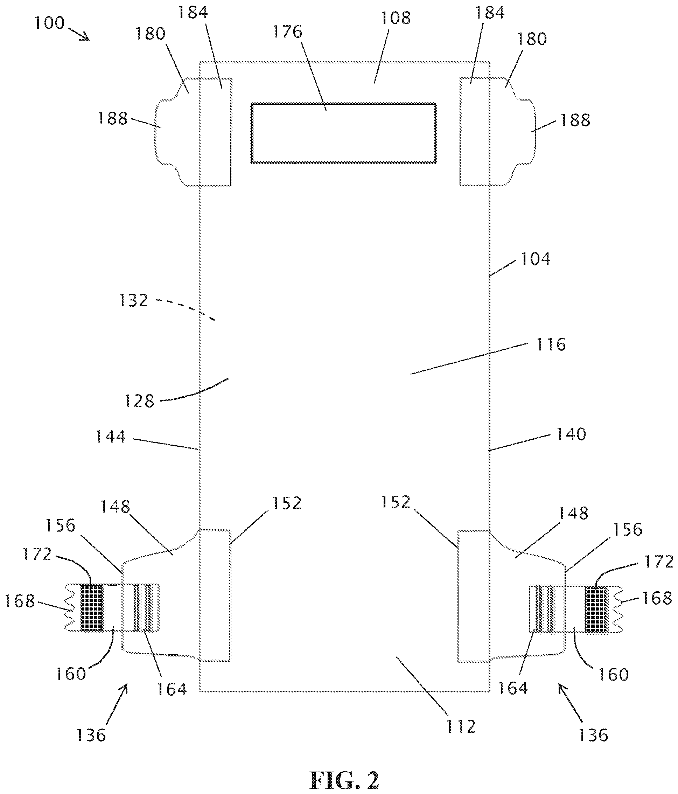

Another example of such a disposable absorbent article is shown in FIG. 2, which depicts a lower plan view of a prior art diaper 100. Diaper 100 includes a chassis 104 having a front waist portion 108, an opposing rear waist portion 112, and a crotch portion 116 extending longitudinally between front and rear waist portions 108, 112. Chassis 104 further includes an outer surface 128 configured to face away from a wearer during use of the diaper, and an opposing body facing surface 132 configured to face a wearer during use of the diaper. In the view of FIG. 2, a dashed leader extends from the body facing surface to reference numeral 132 because body facing surface 132 is opposite outer surface 128 and therefore not visible in the view of FIG. 2.

As shown in FIG. 2, diaper 100 further includes a pair of closure members 136 configured to couple rear waist portion 112 to front waist portion 108 in a well-known configuration in which a left side 140 of the chassis defines a first leg opening for a wearer's left leg, and in which a right side 144 of the chassis defines a second leg opening for the wearer's right leg, similar in some respects to what is shown in FIG. 1B for training pant 10. In the depicted configuration, the closure members include a pair of back ears or back ear panels 148 each having a first end 152 bonded to rear waist portion 112 of chassis 104, and a second end 156 shown extending away from rear waist portion 112. "Bonded" refers to the joining, adhering, connecting, attaching, or the like, of two elements. Two elements will be considered to be bonded together when they are bonded directly to one another or indirectly to one another, such as when each is directly bonded to intermediate elements.

Each closure member 136 further includes a fastener tab 160 with a first end 164 bonded to back ear 148, a second end 168 shown extending laterally outward from back ear 148, and a fastener portion 172 coupled to the fastener tab. Back ears 148 are each formed of a stretchable elastic material, such as a nonwoven laminate, that permits adjustments in the width and tension of back ears 148 to vary the form and fit of diaper 100 when worn by a user. Fastener tabs 160 are formed of an inelastic nonwoven material and carry fastener portions 172. Fastener portions 172 include strips of hook material configured to interact with a corresponding loop material in the well-known hook-and-loop fastener arrangement. Connection of closure members 136 to front waist portion 108 is facilitated by a landing zone 176 configured to be engaged by fastener portions 172. In this embodiment, landing zone 176 is defined by an anchoring member that includes a strip of loop material bonded to front waist portion 108 of chassis 104, for example, to the backsheet, and configured to be engaged by the hook material of fastener portions 172.

As shown in FIG. 2, diaper 100 also includes a pair of front ears 180 extending from opposite sides 140, 144 of chassis 104 with each of front ears 180 each having a first end 184 bonded to front waist portion 108 of chassis 104, and a second end 188 shown extending away from a respective side of front waist portion 108. Front ears 180 are each formed of a relatively soft nonwoven material and are each configured to be overlapped by the corresponding fastener tab 160 and/or back ear 148 to prevent the edges of fastener tab 160 from pinching, rubbing, or otherwise irritating a user's skin in use when fastening portions 172 are engaged with landing zone 176 to couple rear waist portion 112 to front waist portion 108.

Some prior art baby diapers having a configuration similar to that shown in FIG. 2 are provided with a loop fastener material on the back of side of fastener tabs 160, opposite the side of fastener tabs 160 that carry hook fastener portions 172. Such diapers are configured to permit hook fastener portion 172 of one fastener tab 160 to engage the loop material on the back side of the other fastener tab 160 when the fastener tabs 160 overlap one another. In such prior art diapers, however, the hook fastener portion 160 on fastener tab 172 may permit the fastener tabs to rotate relative to one another when the diaper is worn, and thereby permit the front and /or rear waist portions to sag.

Diaper 100 of FIG. 2 is typically packaged and sold in a folded, and unfastened configuration in which chassis 104 is folded in half such that rear waist portion 108 overlaps front waist portion 104, but fastener portions 172 do not engage landing zone 176. Insert page 4a here

### SUMMARY

This disclosure includes embodiments of disposable absorbent articles, assemblies including disposable absorbent articles, and methods of making disposable absorbent articles, for example, training pants and adult pull-up underwear that are adjustable and/or refastenable. For example, some of the present disposable absorbent articles include dual closure members each having a first end coupled to a rear waist portion of the article, and a second end configured to extend away from the rear waist portion and be releasably coupled to the front waist portion. Some such articles include multiple, spaced-apart fastener portions on each closure member to engage the front waist portion. Some of the present assemblies include a plurality of the present articles in a closed configuration in which the closure members are releasably coupled to the front waist portion. Some of the present methods include releasably coupling the closure members to the front waist portion to place the article in a closed configuration, for example, prior to packaging or shipping the article.

Embodiments of the present articles can be configured to provide an adjustable and/or refastenable training pant or pull-up underwear that can be manufactured on existing diaper manufacturing machines. Some such articles include stretchable side panels with extended, over-lapping tabs, each with multiple fasteners for engaging a centered landing zone area in a belt-like manner. Such articles can be made in a pre-fastened state, which is distinct from the open configuration in which diapers are typically distributed to consumers.

WO97/36566 A1 suggest a disposable diaper having elastomeric ear panels and a fastening system that provides the user with different options as to how the absorbent article may be fitted to and removed from the wearer. The diaper allows the wearer to choose between conventional and pull-on diaper configurations, or combinations thereof, and properly and comfortably fit a large range of wearer sizes. Further, the absorbent articles can be pulled on and/or off as a pant. This feature is provided by the ear panels which maintain sufficient tension to hold the diaper on the wearer throughout the period of use without harming the wearer's skin, while providing enough stretch to allow the diaper to be pulled up or down over the wearer's hips.

US5295986A teaches a growth adjustable diaper for newborn babies. The diaper is adjustable in length by use of a foldable absorbent panel at the front edge of the diaper. It is further adjustable about the waist of the baby by means of ear members that can overlap and attach to each other or not overlap and attach to the front section of the diaper.

Suggest manufacturing a disposable absorbent article, by joining a first, second, and third fastener elements to a web. The first fastener element is spaced from the second fastener element by a first distance along a length of the web. The second fastener element is spaced from the third fastener element by a second distance along the length of the web. The second fastener element is disposed between the first and third fastener elements. Subsequently, the first fastener element and the web are cut through at a first point along the length of the web to define a first end of a first fastener panel. Next, the third fastener element and the web are cut through at a second point along the length of the web to define a second end of the first fastener panel. The first fastener panel can now be joined to a chassis of an absorbent article.

The pre-fastened state allows such embodiments to be removed from the retail package and used immediately, by pulling up and onto the torso, like underwear. Refastenable tabs can be adjusted as needed, and multiple fasteners can be provided to allow for secure attachment while in the pre-fastened state, and ease of adjustment when refitting is needed. In some embodiments, graphics may be added to the fastening tabs to allow for a more belt-like or garment like appearance.

Some embodiments of the present disposable absorbent articles comprise: a chassis having opposing front and rear waist portions, a crotch portion extending longitudinally between the front and rear waist portions, a body facing surface configured to face a wearer during use of the article, and an outer surface configured to face away from a wearer during use of the article; and two closure members each having a first end, a second end, and a width extending between the first and second ends, each of the closure members comprising an elastic panel and a plurality of fastener portions coupled to and spaced along a portion of the width of the closure member; where the first end of each closure member is bonded to the rear waist portion of the chassis, and the second end of each closure member is configured to overlap and be releasably coupled to the front waist portion to define a closed configuration in which: the front and rear waist portions cooperate with the closure members to encircle and define a waist opening, a left side of the chassis defines a first leg opening, and a right side of the chassis defines a second leg opening.

In some embodiments of the present disposable absorbent articles, the elastic panel of each closure member has a first end defining the first end of the closure member, a second end, and a width extending between the first and second ends, and each closure member further comprises: a fastener tab having a first end bonded to the elastic panel, and a second end defining a second end of the closure member such that the width of the closure member extends from the first end of the elastic panel to the second end of the fastener tab; where the plurality of fastener portions is spaced along a portion of the width of the fastener tab. In some such embodiments, the fastener portions of each closure member are bonded to or formed on a body facing side of the fastener tab of the closure member, and an outer surface of the fastener tab of a first closure member is configured to be engaged by the fastener portions of the other closure member. In some such embodiments, the fastener tab of the first closure member is configured to overlap the front waist portion with the fastener portions of the first closure member releasably engaged with the front waist portion, and the fastener tab of the other closure member is configured to overlap the first closure member with the fastener portions of the other closure member releasably engaged with the outer surface of the fastener tab of the first closure member.

In some embodiments of the present disposable absorbent articles, the elastic panels of each closure member are bonded directly to a body facing side of the elastic panel of that closure member. In some such embodiments, an outer side of the elastic panel of a first one of the closure members is configured to be engaged by the fastener portions of the other closure member.

In some embodiments of the present disposable absorbent articles, the elastic panel of each closure member has a first end defining the first end of the closure member, a second end, and a width extending between the first and second ends; the chassis has a product height extending from a midpoint of the crotch portion to an outermost edge of the rear waist portion; and a length of the first end of the elastic panel of each closure member, measured parallel to the product height, is greater than or equal to 45% of the product height.

In some embodiments of the present disposable absorbent articles, a landing portion of the outer surface on the front waist portion of the chassis is configured to be releasable engaged by the fastener portions of the closure members. In some such embodiments, the portion of the outer surface is defined by an anchoring member bonded to a backsheet of the chassis. In some such embodiments, the anchoring member comprises a loop material, and the fastener portions of the closure members comprise a hook material configured to releasably engage the loop material.

In some embodiments of the present disposable absorbent articles, the fastener portions of the closure members comprise a hook material.

In some embodiments of the present disposable absorbent articles, the front waist portion, rear waist portion, and crotch portion are defined by corresponding regions of a common chassis member.

In some embodiments of the present disposable absorbent articles, the chassis comprises a topsheet, a backsheet bonded to the topsheet, and an absorbent core disposed between the topsheet and the backsheet.

Some embodiments of the present assemblies comprise: a package; and a plurality of the present disposable absorbent articles disposed in the package with the each of the articles in its closed configuration in which the second ends of its closure members overlap and are releasably coupled to its front waist portion. In such embodiments, the plurality of the present disposable absorbent articles may all be the same, or may include one or more of each of two different embodiments of the present disposable absorbent articles.

In some embodiments of the present methods of manufacturing a disposable absorbent article having a chassis with opposing front and rear waist portions, a crotch portion extending longitudinally between the front and rear waist portions, and a body facing surface configured to face a wearer during use of the article, the method comprises: providing an embodiment of the present disposable absorbent articles; releasably coupling the second ends of the closure members of the article to the front waist portion of the article such that the second ends of the closure members overlap the front waist portion in the closed configuration; and disposing the article in the closed configuration in a package for distribution.

In some embodiments of the present methods, the elastic panel of each closure member has a first end defining the first end of the closure member, a second end, and a width extending between the first and second ends, and each closure member further comprises: a fastener tab having a first end bonded to the elastic panel, and a second end defining a second end of the closure member such that the width of the closure member extends from the first end of the elastic panel to the second end of the fastener tab; where the plurality of fastener portions is spaced along a portion of the width of the fastener tab; and where the fastener portions of each closure member are bonded to or formed on a body facing side of the fastener tab, and an outer surface of the fastener tab of a first closure member is configured to be engaged by the fastener portions of the other closure member. In some such embodiments, releasably coupling the second ends of the closure members comprises releasably coupling the fastener portions of the first closure member to the front waist portion of the chassis, and releasably coupling at least one of the fastener portions of the other closure member to the outer side of the fastener tab of the first closure member.

In some embodiments of the present methods, the elastic panels of each closure member are bonded directly to a body facing side of the elastic panel of that closure member, and coupling the second ends of the closure members comprises releasably coupling the fasteners of each closure member to the front waist portion such that the second ends of the closure members do not overlap.

In some embodiments of the present methods, the elastic panels of each closure member are bonded directly to a body facing side of the elastic panel of that closure member, an outer side of the elastic panel of a first one the closure members is configured to be engaged by the fastener portions of the other closure member, and coupling the second ends of the closure members comprises releasably coupling the fastener portions of the first closure member to the front waist portion of the chassis, and releasably coupling at least one of the fastener portions of the other closure member to the outer side of the elastic panel of the first closure member.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the term "substantially" may be substituted with "within [a percentage] of' what is specified, where the percentage includes 0.1, 1, 5, and 10 percent.

The terms "comprise" and any form thereof such as "comprises" and "comprising," "have" and any form thereof such as "has" and "having," and "include" and any form thereof such as "includes" and "including" are open-ended linking verbs. As a result, an apparatus that "comprises," "has," or "includes" one or more elements possesses those one or more elements, but is not limited to possessing only those elements. Likewise, a method that "comprises," "has," or "includes" one or more steps possesses those one or more steps, but is not limited to possessing only those one or more steps.

Any embodiment of any of the apparatuses, systems, and methods can consist of or consist essentially of - rather than comprise/include/have - any of the described steps, elements, and/or features. Thus, in any of the claims, the term "consisting of' or "consisting essentially of' can be substituted for any of the open-ended linking verbs recited above, in order to change the scope of a given claim from what it would otherwise be using the open-ended linking verb.

Further, a device or system that is configured in a certain way is configured in at least that way, but it can also be configured in other ways than those specifically described.

The feature or features of one embodiment may be applied to other embodiments, even though not described or illustrated, unless expressly prohibited by this disclosure or the nature of the embodiments.

Some details associated with the embodiments described above and others are described below.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. Views in the figures are drawn to scale, unless otherwise noted, meaning the sizes of the depicted elements are accurate relative to each other for at least the embodiment in the view.
**FIG. 1A** depicts a bottom plan view of a prior art disposable absorbent article, specifically a training pant, in an open configuration.
**FIG. 1B** depicts a perspective view of the training pant of FIG. 1A in a closed configuration.
**FIG. 2** depicts a bottom plan view of a prior art disposable absorbent article, specifically a diaper, in an open configuration.
**FIG. 3** depicts a bottom plan view of a first embodiment of the present disposable absorbent articles, specifically a training pant, in an open configuration.
**FIGS. 4A-4C** depicts front plan views of the training pant of FIG. 3 showing a progression of folds to convert the training pant from its open configuration to a closed configuration shown in FIG. 4C.
**FIG. 5A** depicts a plan view of a second embodiment of the present disposable absorbent articles, specifically a training pant, in an open configuration.
**FIG. 5B** depicts a plan view of a variation of fastener portion spacing on a fastener tab suitable for at least some embodiments of the present absorbent articles.
**FIGS. 6A-6C** depicts front plan views of a third embodiment of the present disposable absorbent articles, specifically a training pant, showing a progression of folds to convert the training pant from an open configuration to a closed configuration shown in FIG. 6C.
**FIG. 7** depicts a top plan view of a package containing a plurality of the present absorbent articles.

### DETAILED DESCRIPTION OF ILLUSTRATIVE EMBODIMENTS

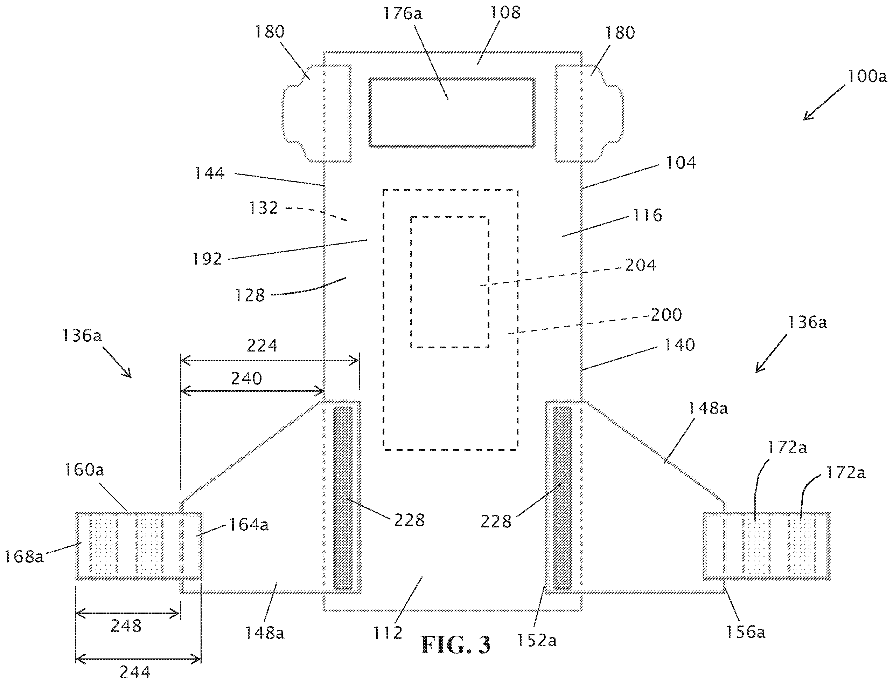

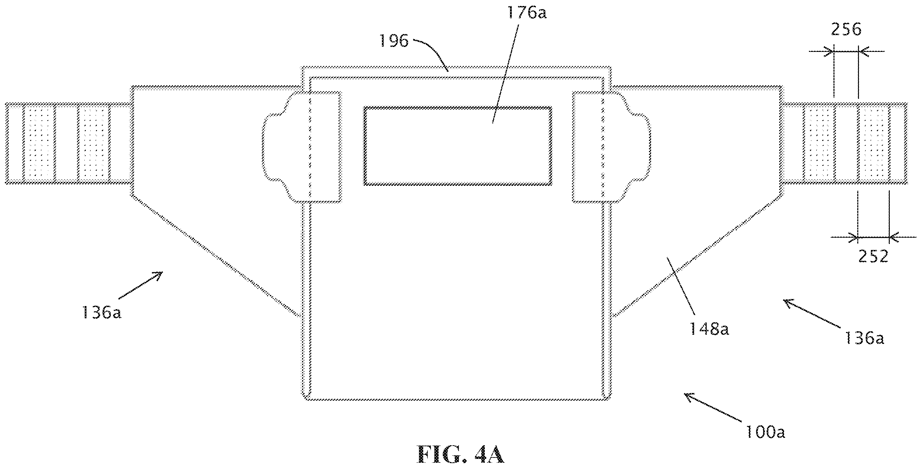

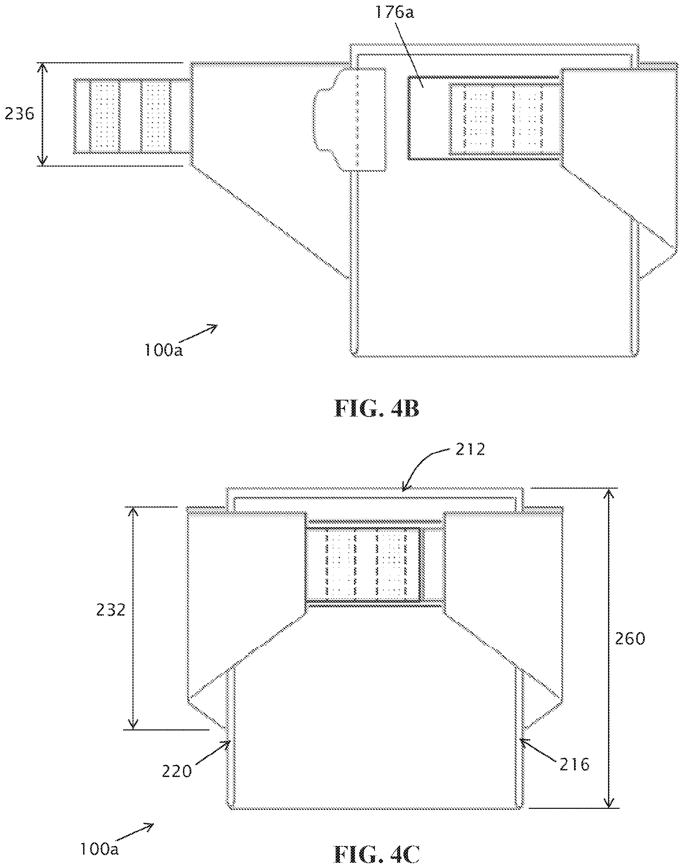

Referring now to FIGS. 3 and 4A-4C, FIG. 3 depicts a bottom plan view of a first embodiment 100a of the present disposable absorbent articles in an open and unfastened configuration; and FIGS. 4A-4C depict front plan views of article 100a in various stages showing a progression of folds to convert the article from its open configuration of FIG. 3 to a closed and fastened configuration shown in FIG. 4C. Article 100a is similar in some respects to article 100. For example, although the depicted embodiment of article 100a is a training pant rather than a diaper, article 100a includes a chassis 104 having a front waist portion 108, an opposing rear waist portion 112, and a crotch portion 116 extending longitudinally between front and rear waist portions 108, 112. Chassis 104 further includes an outer surface 128 configured to face away from a wearer during use of the training pant, and an opposing body facing surface 132 configured to face a wearer during use of the training pant. More particularly, training pant 100a includes a liquid-impermeable backsheet or cover 192 that defines outer surface 128, and a liquid-permeable topsheet 196 that defines body facing surface 132 and is configured to be closest to the wearer during use. "Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. "Lamination" is the technique of manufacturing a material in multiple layers, so that the composite material has benefits of all the combined layers, such as, for example, improved mechanical strength or durability, improved stability, lower permeability to water, and/or other properties. A laminate includes two or more layers of material(s) that are a permanently assembled by heat, pressure, welding, or adhesives.

Liquid-impermeable backsheet 192 can include, for example, an inner liquid-impermeable film and an outer nonwoven backsheet that can be a nonwoven fabric. A "film" is a membrane-like layer of material formed of one or more polymers, which does not have a form consisting predominately of a web-like structure of fibers and/or other fibers. In some embodiments of the present articles, backsheet or outer cover 192 can be breathable, for example, an inner liquid-impermeable film of backsheet 192 can comprise a breathable film. The terms "breathable," "breathable film," "breathable laminate" or "breathable outer cover material" or "breathable backsheet" refers to a film, laminate, or outer cover material having a water vapor transmission rate ("WVTR") of at least about 300 grams/m²/24 hours. Breathable materials typically rely on molecular diffusion of vapor, and are substantially liquid impermeable. "Nonwoven" fabrics, according to an INDA definition, are broadly defined as sheet or web structures bonded together by entangling fiber or filaments (and by perforating films) mechanically, thermally, or chemically. They are flat, porous sheets that are made directly from separate fibers or from molten plastic or plastic film. They are not made by weaving or knitting and do not require converting the fibers to yarn. The basis weight of nonwoven fabrics is usually expressed as gsm or grams per square meter. "Nonwoven backsheet" is a backing substrate layer in the outer cover; a nonwoven backsheet is most often a nonwoven layer facing away from the wearer.

An absorbent core 200 is disposed between topsheet 196 and backsheet 192. An "absorbent core" is a structure typically disposed between a topsheet and backsheet of an absorbent article and containing materials like SAP and/or cellulosic fibers that are configured to absorb liquid in the absorbent article. The absorbent core may also include a cover layer or envelope material. The absorbent core, and/or the cover layer or envelope material, may comprise; nonwovens, SAP, cellulosic or non-cellulosic materials, films, fibers, or one or more substrates made of any one two or all of these combination materials. "Superabsorbent" or "superabsorbent material" or "SAP" refers to a water-swellable, water-insoluble organic or inorganic material capable, under the most favorable conditions, of absorbing at least about 15 times its weight in an aqueous solution containing 0.9 weight percent sodium chloride and, more desirably, at least about 30 times its weight in an aqueous solution containing 0.9 weight percent sodium chloride and, even more desirably, at least about 50 times its weight in an aqueous solution containing 0.9 weight percent sodium chloride. The SAP materials can be natural, synthetic and modified natural polymers and materials. In addition, the SAP materials can be inorganic materials, such as silica gels, or organic compounds such as cross linked polymers.

In the embodiment shown in FIGS. 3 and 4A-4C, training pant 100a also includes an acquisition-distribution layer (ADL) 204 disposed between the topsheet and the absorbent core. "Layer" when used in the singular can be a single element or a plurality of elements. For example, a plurality of sheets may together define a single layer, such as, for example, a layer with a particular function to which the sheets of the layer contribute. Chassis 104 is shown as a single-member chassis in which front waist portion 108, rear waist portion 112, and crotch portion 116 are defined by corresponding regions of a common chassis member. However, in other embodiments, the present absorbent articles can include a multi-member chassis, such as, for example, a well-known three-part construction in which the front waist portion is defined by a first chassis member, the rear waist portion is defined by a second chassis member, and the crotch portion is defined by a third chassis member having a first end coupled to the first chassis member and a second end coupled to the second chassis member.

As with diaper 100, training pant 100a includes a pair of symmetrical closure members 136a configured to couple rear waist portion 112 to front waist portion 108 in a well-known configuration in which front waist portion 108, rear waist portion 112, and closure members 136a encircle and define a waist opening 212; in which a left side 140 of the chassis defines a first leg opening 216 for a wearer's left leg; and in which a right side 144 of the chassis defines a second leg opening 220 for the wearer's right leg. In contrast with diaper 100, however, the closure members of training pant 100a each comprise an enlarged back ear panel 148a. In the embodiment shown, back ear panels 148a are each formed of a stretchable elastic material, such as a nonwoven laminate, that permits adjustments in the width and tension of back ears 148a to vary the form and fit of training pant 100a when worn by a user. As used herein and in the claims, the term "stretch" and "stretchable" are used interchangeably to define a material or composite that can be elongated by at least 25% of its relaxed dimension, i.e., elongated to at least 1 ¼ times its relaxed dimension (an elongation of 25%), and that will recover upon release of the applied force at least 10% of its elongation. According to this definition, upon release of the applied force at 25% elongation, the material or composite must recover to at least about a 15% or less elongation. For example, a material or composite is deemed to be "stretchable" if a sample length of 100 centimeters can be elongated to a length of at least 125 centimeters, and upon release of the applied force recovers to a length of not more than about 115 centimeters. Many elastic or stretchable materials or composites can be elongated by more than 25% of their relaxed length, and many of these will recover to, or close to, their original relaxed length upon release of the applied force. These materials can include not only webs of elastic or stretchable films, such as cast or blown films, but also nonwoven fibrous elastic webs such as meltblown elastomeric fibrous nonwoven webs and elastic strands. "Elastic," "elasticized" and "elasticity" mean that property of a material by virtue of which it tends to recover its original size and shape after removal of a force causing a deformation.

Closure members 136a may, for example, be more similar in size and function to side panels 38, 42 of training pant 10 than to closure members 136 of diaper 100. In the embodiment shown, closure members 136a are symmetrical, and each closure member 136a includes an enlarged back ear or back ear panel 148a having a first end 152a bonded to rear waist portion 112 of chassis 104, a second end 156a shown extending away from rear waist portion 112, and a width 224 extending between the outermost edges of first and second ends 152a, 156a. For example, in the embodiment shown, first end 152a of each back ear panel 148a is bonded to rear waist portion 112 via an adhesive bond 228. In other embodiments, bond 228 can be or include a thermal bond, ultrasonic bond, and/or any other type of bond that secures the respective closure member to the chassis 104 with sufficient strength for training pant 100a to function as described in this disclosure.

In the embodiment shown in FIGS. 3 and 4A-4C, each closure member 136a has a width that is the sum of widths 240 and 248 and that is sufficient, for users of expected sizes or waist dimensions, to wrap around a portion of the user's waist and engage front waist portion 108. As shown, in this embodiment, each back ear panel 148a further has a first length 232 at its first end 152a, and a second length 236 at its second end 156a. As shown, second length 236 may be smaller than first length 232 such that each closure member is tapered along its width. For example, as shown in FIGS. 4B and 4C, the length of each closure member decreases linearly along width 224 from length 232 at first end 152a to length 236 at second end 156a. In other embodiments, each closure member 136a may have a first length that is equal to its second length such that the closure member has a constant length along its width 224.

In the embodiment shown in FIGS. 3 and 4A-4C, each closure member 136a also includes an enlarged fastener tab 160a with a first end 164a bonded to back ear 148a, a second end 168a shown extending laterally outward from back ear 148a, and a plurality of fastener portions 172a coupled to the fastener tab. Fastener tabs 160a may, for example, comprise or be formed of an inelastic nonwoven material. In this embodiment, fastener portions 172a include strips or areas of hook material configured to interact with loops or fibers of a corresponding material in the well-known hook-and-loop fastener arrangement. Connection of closure members 136a to front waist portion 108 is facilitated by a landing zone 176a configured to be engaged by fastener portions 172a. As shown, each fastener tab 160a has a width 244 extending between its first and second ends 164a, 168a, and a free width 248 extending beyond the edge of the corresponding back ear panel 148a. In this embodiment, landing zone 176a is defined by an anchoring member that comprises a panel of loop material that is bonded to front waist portion 108 of chassis 104, for example, to backsheet 192, and configured to be engaged by the fastener portions 172a of each closure member. In other embodiments, landing zone 172a may instead be defined by the outermost layer of material of the chassis if the material has fibers that are engage-able by a hook fastener material.

For purposes of this disclosure, the dimensions of an elastic element, such as back ear panel 148a, is measured with the closure member flat against a planar surface and in a relaxed configuration in which the web of material is in its longest unstretched configuration, *i.e.,* is not elastically deformed. Each back ear panel 148a is therefore measured in an extended but relaxed configuration in which the closure member has been elongated enough to remove any noticeable slack, but has not been elongated so much that it elastically deforms. The width or the length of such a back ear panel may therefore each be considered a "relaxed" width or "relaxed" length, respectively, in that it is measured without placing the back ear panel 148a in a stretched state. In some embodiments, such as for a newborn infant, width 224 of each back ear panel 148a is greater than or equal to 65 mm. In other embodiments, width 224 may be larger to accommodate toddlers, youths, or adults. For example, width 224 of each back ear panel 148a can be greater than or equal to any one of, or between any two of: 55, 65, 75, 85, 100, 125, 150, 175, and/or 200, or more millimeters (mm). In some embodiments, a free width 240 of each back ear panel 148a is defined as the portion of the back ear panel that extends beyond the respective side or edge, 140 or 144, of chassis 104. In some embodiments, free width 240 of each back ear panel 148a is equal to width 224 less the margin along which back ear panel 148a is bonded to the rear waist portion of the chassis; free width 240 may, for example, be greater than or equal to 55 mm in garments sized for a newborn infant. In other embodiments, free width 240 may be larger to accommodate toddlers, youths, or adults. For example, free width 240 of each back ear panel 148a can be greater than or equal to any one of, or between any two of: 45, 55, 65, 75, 90, 115, 140, 165, and/or 190, and/or more millimeters (mm).

In some embodiments, such as for a newborn infant, a width 244 of each fastener tab 160a is greater than or equal to 60 mm. In other embodiments, width 244 may be larger to accommodate toddlers, youths, or adults. For example, width 244 of each fastener tab 160a can be greater than or equal to any one of, or between any two of: 50, 60, 70, 80, 90, 100, 125, 150, 175, 200, and/or more millimeters (mm). In some embodiments, a free width 248 of each fastener tab 160a is equal to width 244 less the margin along which fastener tab 160a is bonded to the back ear panel; free width 240 may, for example, be greater than or equal to 50 mm in garments sized for a newborn infant. In other embodiments, free width 248 may be larger to accommodate toddlers, youths, or adults. For example, free width 248 of each fastener tab 160a can be greater than or equal to any one of, or between any two of: 40, 50, 60, 70, 80, 90, 115, 140, 165, 190, and/or more millimeters (mm). In some embodiments, such as for a newborn infant, first length 232 of each back ear panel 148a may be greater than or equal to 80 mm, and/or second length 236 may be greater than or equal to 50 mm.

In the embodiment shown in FIGS. 3 and 4A-4C, each fastener portion 172a has a width 252 measured parallel to width 224 of the corresponding one of back ear panels 148a. For example, in some embodiments, width 252 is greater than or equal to any one of, or between any two of: 2, 4, 6, 8, 10, 12, 14, 16, 18, 20, 22, 24, 26 or more millimeters (mm). In some embodiments, fastener portions 172a on a single fastener tab 160a are separated by a distance 256 that is at least 50% of the width 252 of one individual fastener portion 172a. For example, in some embodiments, the fastener portions 172a on a fastener tab 160 may be separated by a distance 256 of greater than or equal to any one of, or between any two of: 50%, 60%, 70%, 80%, 90%, 100%, 150%, 200%, 250%, 300%, 350%, 400%, or more of width 252. In some embodiments, fastener portions 172a are formed in-line during manufacture of the training pant 100a, rather than being separately formed and attached as a separate sheet of material. For example, U.S. Patent No. 8,784,722 discloses a method and apparatus for producing hook fasteners.

Training pant 100a may be said to have a product height 260 of roughly one half of the overall length of the chassis when chassis 104 is folded along a midpoint between the outermost edge of front waist portion 108, and the outermost edge of rear waist portion 112, as shown in FIG. 4A. In the embodiment shown, length 232 of the first end of each back ear 148a is greater than or equal to 45% of product height 260. In some embodiments, length 232 of the first end of each back ear 148a is greater than or equal to any one of, or between any two of, 40%, 50%, 60%, 70%, 80%, 90%, or more of product height 260.

Chassis 104 may then be folded along a midpoint between the outermost edge of front waist portion 108, and the outermost edge of rear waist portion 112, as shown in FIG. 4A.

In embodiment shown in FIGS. 3 and 4A-4C, closure members 136a are configured to be releasably coupled or engaged to front waist portion 108 such that front and rear waist portions 108, 112 cooperate with closure members 136a to encircle and define a waist opening 212, left side 140 of chassis 104 defines a first leg opening 216, and right side 144 of chassis 104 defines a second leg opening 220. More particularly, and as shown in FIG. 4C, the width of each closure member 136a is sufficiently large for the closure members to be wrapped around a portion of front waist portion 108 with one of the closure members overlapping the other of the closure members. As shown in FIG. 3, training pant 100a may begin in an unfolded configuration. Chassis 104 may then be folded along a midpoint between the outermost edge of front waist portion 108, and the outermost edge of rear waist portion 112, as shown in FIG. 4A. From the position of FIG. 4A, a first one of the closure members can be folded over front waist portion 108 such that that fastener members of the first closure member releasably engages landing zone 176a, as shown in FIG. 4B. From the position of FIG. 4B, a second one of the closure members can then be folded over front waist portion 108. In this configuration, an outer surface of fastener tab 160a of at least the first closure member 136a can be configured to be releasably engaged by fastener portions 172a of the other closure member 136a, such that the second closure member 136a can be folded over front waist portion 108 and the fastener portions of the second closure releasably engage landing zone 176a as shown in the closed configuration of FIG. 4C. Such embodiments may be folded, and the fastener portions secured to landing zone 176a, prior to the article or training pant 100a being inserted into a package, for example, for distribution or sale.

As with diaper 100, training pant 100a includes a pair of front ears 180 extending from opposite sides 140, 144 of chassis 104. In this embodiment, front ears 180 are substantially similar to those of diaper 100. In other embodiments of diaper 100, training pant 100a, and/or others of the present disposable absorbent articles, front ears 180 are optional and may be omitted. In some embodiments, front ears 180 include loop fastener portions or a fabric that is configured to be engaged by hook fastener portions such that fastener portions 172a can engage front ears 180.

In this disclosure, the term "width" is typically used to refer to dimensions that will lie along the circumference of a user's waist when the present garments are worn by the user, and the term "length" is used to refer to dimensions that will lie at an angle, such as a perpendicular angle, relative to the user's waist when the present garments are worn. As such, the term "length" does not necessarily indicate the longest dimension of an element. For example, width 224 of back ear panel 148a may be longer than its length 232.

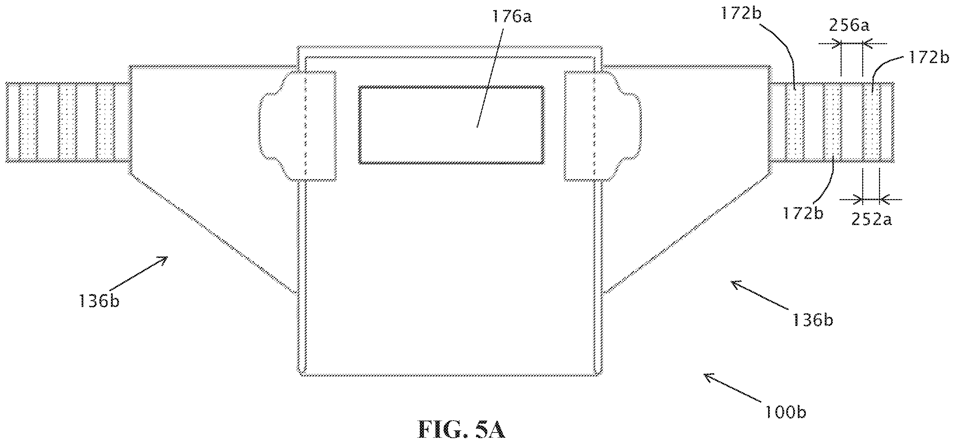

FIG. 5A depicts a plan view of a second embodiment of the present disposable absorbent articles 100b. Article 100b is a training pant that is similar in many respects to training pant 100a, and similar reference numbers are used to designate features of article 100b that are similar to correspondingly numbered features in article 100a. Because article 100b is so similar to article 100a, the differences will primarily be described here. For example, each closure member 136b of article 100b includes three fastener portions 172b spaced apart by a distance 256a. In this embodiment, each fastener portion 172b has a width 252a measured parallel to the width (224) of the corresponding one of back ear panels 148a, and the fastener portions on a single fastener tab 160a are separated by a distance 256a that is at least 50% of the width 252a of one individual fastener portion 172b. In some embodiments, such as for a newborn infant, width 252a is 2 mm. In other embodiments, width 252a may be larger to accommodate toddlers, youths, or adults. For example, width 252a may be greater than or equal to any one of, or between any two of: 2, 4, 6, 8, 10, 12, 14, 16, 18, 20, 22, 24, 26 or more millimeters (mm). By way of further example, in some embodiments, the fastener portions 172b on a fastener tab 160a may be separated by a distance 256a of greater than or equal to any one of, or between any two of: 50%, 60%, 70%, 80%, 90%, 100%, 150%, 200%, 250%, 300%, 350%, 400%, or more of width 252a.

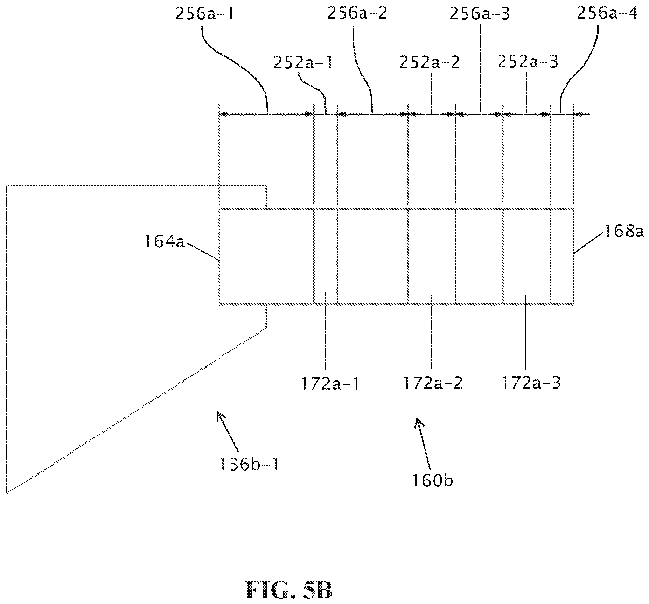

FIG. 5B depicts a plan view of a variation of fastener portion spacing on a fastener tab 160b that is suitable for inclusion in a closure member 136b-1 in at least some embodiments of the present absorbent articles. Fastener tab 160b is similar in many respects to fastener tab 160a, and similar reference numbers are used to designate features of fastener tab 160b that are similar to correspondingly numbered features in fastener tab 160a. In this embodiment, fastener tab 160b includes three fastener portions 172b-1, 172b-2, 172b-3 with widths that vary, and spacing between fastener portions that vary. In the particular configuration shown in FIG. 5B, fastener portion 172b-1 has a width 252a-1, fastener portion 172b-2 has a width 252a-2 that is twice width 252a-1, and fastener portion 172b-3 has a width 252a-3 that is equal to width 252a-2. In this particular configuration, fastener portion 172b-1 is spaced from first or inner end 164a of fastener tab 160b by a distance 256a-1 that is four times width 252a-1, fastener portion 172b-2 is spaced from fastener portion 172b-1 by a distance 256a-2 that is three times width 252a-1, fastener portion 172b-3 is spaced from fastener portion 172b-1 by a distance 256a-3 that is two times width 252a-1, and fastener portion 172b-3 is spaced from second or outer end 168a of fastener tab 160b by a distance that is equal to width 252a-1. In some such embodiments, such as for a newborn infant, width 252a-1 is 2 mm. In other embodiments, width 252a-1 may be larger to accommodate toddlers, youths, or adults. For example, width 252a-1 may be greater than or equal to any one of, or between any two of: 2, 4, 6, 8, 10, 12, 14, or more millimeters (mm). In other configurations, the relative sizes of widths 262a-1, 252a-2, and 252a-3 may be varied; for example, width 252a-3 may be larger than width 252a-2 and/or width 252a-2 may be equal to width 252a-1. Likewise, the relative sizes of distances 256a-1, 256a-2, 256a-3, and 256a-4 may be varied; for example, distance 256a-2 may be equal to distance 256a-3 and/or 256a-1.

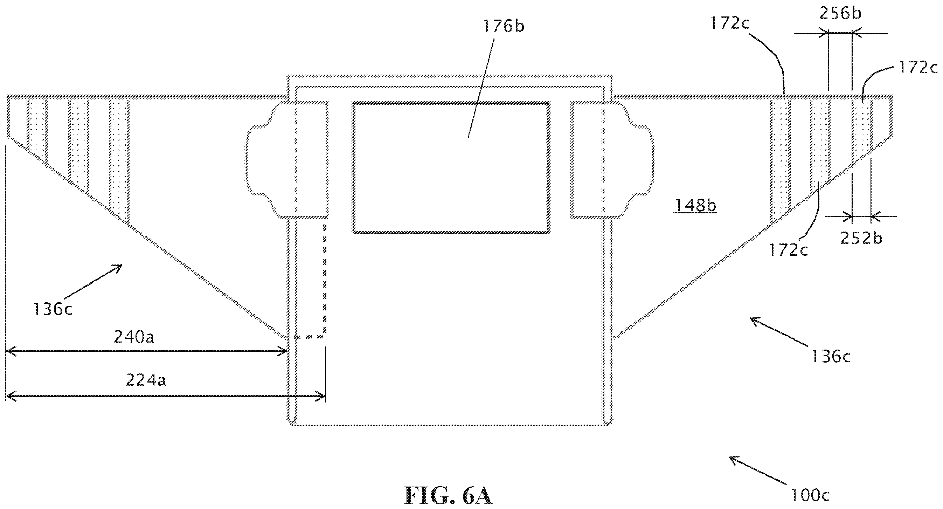

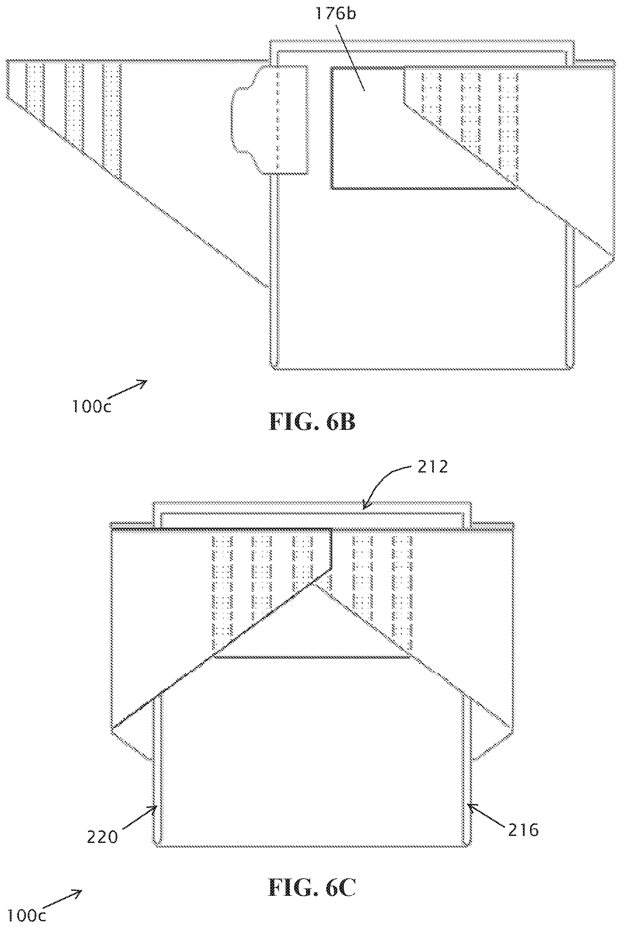

FIGS. 6A-6C depict front plan views of a third embodiment 100c of the present disposable absorbent articles, showing a progression of folds to convert the training pant from an open configuration to a closed configuration shown in FIG. 6C. Article 100c is a training pant that is similar in many respects to training pant 100a, and similar reference numbers are used to designate features of article 100c that are similar to correspondingly numbered features in article 100a. Because article 100c is so similar to article 100a, the differences will primarily be described here. For example, each closure member 136c of article 100c includes three fastener portions 172c spaced apart by a distance 256b. In this embodiment, each fastener portion 172c has a width 252b measured parallel to width 224a of the corresponding one of back ear panels 148b, and the fastener portions are bonded directly to the back ear panel rather than to a fastener tab. As shown, fastener panels 172c on a single back ear panel are separated by a distance 256b that is at least 50% of the width 252b of one individual fastener portion 172c. For example, in some embodiments, the fastener portions 172c on a back ear panel 148b may be separated by a distance 256b of greater than or equal to any one of, or between any two of: 50%, 60%, 70%, 80%, 90%, 100% or more of width 252. In this embodiment, landing zone 176b is enlarged relative to landing zone 176a to provide additional area for fastener portions 172 to the engage front waist portion of the chassis. While landing zone 176b may comprise a portion of loop material, as with landing zone 176a, landing zone 17b may instead be defined by the outermost layer of material of the chassis if the material has fibers that are engage-able by a hook fastener material.

In the absence of fastener tabs, back ear panels 148b of article 100c may be longer than back ear panels 148a. In some embodiments, such as for a newborn infant, width 224a of each back ear panel 148b is greater than or equal to 100 mm. In other embodiments, width 224a may be larger to accommodate toddlers, youths, or adults. For example, width 224a may be greater than or equal to any one of, or between any two of: 100, 110, 120, 130, 140, 150, 165, 180, 200, 225, 250, or more millimeters (mm). In some embodiments, a free width 240a of each back ear panel 148b is defined as the portion of the back ear panel that extends beyond the respective side or edge, 140 or 144, of chassis 104. In some embodiments, free width 240a of each back ear panel 148b is equal to width 224a less the margin along which back ear panel 148b is bonded to the rear waist portion of the chassis; free width 240a may, for example, be greater than or equal to 90 mm in garments sized for a newborn infant. In other embodiments, free width 240a may be larger to accommodate toddlers, youths, or adults. For example, free width 240a of each back ear panel 148a can be greater than or equal to any one of, or between any two of: 90, 100, 110, 120, 130, 140, 155, 170, 190, 215, 240, or more millimeters (mm).

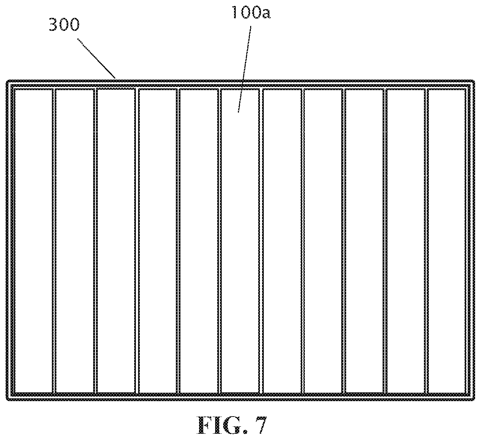

FIG. 7 depicts a top plan view of a packaging containing a plurality of the present absorbent articles 100a disposed in and enclosed by a package 300. In the embodiment shown, articles 100a are in a closed or pre-fastened configuration in which the fastener portions of the closures members of each article have been and are engaged with the landing zone 176a such that a user can remove an article from the package and simply pull it on like a textile garment. Package 300 can comprise, for example, a plastic bag and/or cardboard box. In some embodiments, the articles are disposed in the package in a compressed state that is retained by the package.

The above specification and examples provide a complete description of the structure and use of illustrative embodiments. Although certain embodiments have been described above with a certain degree of particularity, or with reference to one or more individual embodiments, those skilled in the art could make numerous alterations to the disclosed embodiments without departing from the scope of this invention. As such, the various illustrative embodiments of the methods and systems are not intended to be limited to the particular forms disclosed. Rather, they include all modifications and alternatives falling within the scope of the claims, and embodiments other than the one shown may include some or all of the features of the depicted embodiment. For example, elements may be omitted or combined as a unitary structure, and/or connections may be substituted. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several embodiments.

The claims are not intended to include, and should not be interpreted to include, means-plus- or step-plus-function limitations, unless such a limitation is explicitly recited in a given claim using the phrase(s) "means for" or "step for," respectively.

## Claims

1. A disposable absorbent article (100, 100a) comprising a chassis (104) having:
- opposing front and rear waist portions (108, 112),
- a crotch portion (116) extending longitudinally between the front and rear waist portions (108, 112),
- a body facing surface (132) configured to face a wearer during use of the article (100, 100a),
- an outer surface (128) configured to face away from a wearer during use of the article (100, 100a),
- a first closure member (136) and a second closure member (136a) each closure member having a first end (152a), a second end (156a), and a width extending between the first and second ends, each of the closure members (136, 136a) comprising an elastic panel and a plurality of fastener portions (172, 172a) coupled to and spaced along a portion of the width of the closure member (136, 136a),
**characterized in that**:
- the first end (152a) of each closure member (136, 136a) is bonded to the rear waist portion (112) of the chassis (104), and the second end (156a) of each closure member (136, 136a) is configured to overlap and be releasably coupled to the front waist portion (108) to define a closed configuration in which:
- the front and rear waist portions (108, 112) cooperate with the closure members (136, 136a) to encircle and define a waist opening,
- a left side of the chassis (104) defines a first leg opening (216), and
- a right side of the chassis (104) defines a second leg opening (220),
the plurality of fastener portions (172, 172a) of the first closure member (136, 136a) are configured to overlap the front waist portion (108) with the fastener portions (172, 172a) of the first closure member (136, 136a) releasably engaged with the front waist portion (108), and
the plurality of fastener portions (172, 172a) of the second closure member (136, 136a) are configured to overlap the first closure member (136, 136a) with the fastener portions (172, 172a) of the second closure member (136, 136a) releasably engaged with the outer surface of the fastener portions (172, 172a) of the first closure member (136, 136a).

2. The article of claim 1, **characterized in that** the elastic panel of each closure member (136, 136a) has a first end defining the first end (152a) of the closure member (136, 136a), a second end, and a width extending between the first and second ends, and each closure member (136, 136a) further comprises:
- a fastener tab (160, 160a) having a first end (164) bonded to the elastic panel, and a second end (168) defining a second end (156a) of the closure member such that the width of the closure member (136, 136a) extends from the first end of the elastic panel to the second end of the fastener tab (160, 160a);
- where the plurality of fastener portions (172, 172a) is spaced along a portion of the width of the fastener tab (160, 160a).

3. The article of claim 2, **characterized in that** the fastener portions (172, 172a) of each closure member (136, 136a) are bonded to or formed on a body facing side of the fastener tab (160, 160a) of the closure member (136, 136a), and an outer surface of the fastener tab (160, 160a) of the first closure member (136, 136a) is configured to be engaged by the fastener portions (172, 172a) of the second closure member (136, 136a).

4. The article of claim 3, **characterized in that** the fastener tab (160, 160a) of the first closure member (136, 136a) is configured to overlap the front waist portion (108) with the fastener portions (172, 172a) of the first closure member releasably engaged with a landing zone (176, 176a) of the front waist portion (108), and the fastener tab (160, 160a) of the second closure member (136, 136a) is configured to overlap the first closure member with the fastener portions (172, 172a) of the second closure member (136, 136a) releasably engaged with both the landing zone (176, 176a) and the outer surface of the fastener tab (160, 160a) of the first closure member (136, 136a).

5. The article of claim 1, **characterized in that** the elastic panels of each closure member (136, 136a)
- are bonded directly to a body facing side of the elastic panel of that closure member; or
- are bonded directly to a body facing side of the elastic panel of that closure member (136, 136a) and **in that** an outer side of the elastic panel of a first one of the closure members (136, 136a) is configured to be engaged by the fastener portions (172, 172a) of the second closure member (136, 136a); or
- have a first end defining the first end (152a) of the closure member (136, 136a), a second end, and a width extending between the first and second ends; the chassis (104) has a product height extending from a midpoint of the crotch portion to an outermost edge of the rear waist portion (112); and a length of the first end of the elastic panel of each closure member (136, 136a), measured parallel to the product height, is greater than or equal to 45% of the product height.

6. The article of claim 1, **characterized in that** a landing portion (176, 176a, 176b) of the outer surface on the front waist portion (108) of the chassis (104) is configured to be releasable engaged by the fastener portions (172, 172a) of the closure members (136, 136a), the plurality of fastener portions (172, 172a, 172b) of the second closure member (136, 136a) includes a first fastener and a second fastener, and the second closure member (136, 136a) is configured to be folded over front waist portion (108) such that the first fastener is releasably engaged with the outer surface of the first closure member (136, 136a) and the second fastener is releasably engaged with the landing portion (176, 176a, 176b).

7. The article of claim 6, **characterized in that** the landing portion (176, 176a, 176b) of the outer surface is defined by an anchoring member bonded to a backsheet of the chassis (104).

8. The article of claim 7, **characterized in that** the anchoring member comprises a loop material, and the fastener portions (172, 172a) of the closure members (136, 136a) comprise a hook material configured to releasably engage the loop material.

9. The article of any of claims 1-8, **characterized in that** the fastener portions (172, 172a) of the closure members (136, 136a) comprise a hook material, and wherein each fastener portion (172, 172a) of the plurality of fastener portions (172, 172a) of the second closure member (136, 136a) is separated by a first distance (256) that is at least 50% of a width of at least one of the respective fastener portions (172, 172a).

10. The article of any of claims 1-9, **characterized in that**
- The front waist portion (108), rear waist portion (112), and crotch portion are defined by corresponding regions of a common chassis (104) member; or
- the chassis (104) comprises a topsheet, a backsheet bonded to the topsheet, and an absorbent core disposed between the topsheet and the backsheet.

11. An assembly comprising:
- a plurality of disposable absorbent articles of any one of claims 1 to 10 and
- a package; and
**characterized in that** the plurality of disposable absorbent articles is disposed in the package with the each of the articles in its closed configuration in which the second ends (156a) of its closure members (136, 136a) overlap and are releasably coupled to its front waist portion (108).

12. A method of packaging a disposable absorbent article having a chassis (104) with opposing front and rear waist portions (108, 112), a crotch portion extending longitudinally between the front and rear waist portions (108, 112), and a body facing surface configured to face a wearer during use of the article, **characterized in that** the method comprises:
- providing a disposable absorbent article of claim 1;
- releasably coupling the second ends (156a) of the closure members (136, 136a) of the article to the front waist portion (108) of the article such that the second ends (156a) of the closure members (136, 136a) overlap the front waist portion (108) in the closed configuration; and disposing the article in the closed configuration in a package for distribution.

13. The method of claim 12, **characterized in that** the elastic panel of each closure member (136, 136a) has a first end (152a) defining the first end (152a) of the closure member (136, 136a), a second end, and a width extending between the first and second ends, and each closure member (136, 136a) further comprises:
- a fastener tab (160, 160a) having a first end bonded to the elastic panel, and a second end defining a second end (156a) of the closure member (136, 136a) such that the width of the closure member (136, 136a) extends from the first end of the elastic panel to the second end of the fastener tab (160, 160a);
- where the plurality of fastener portions (172, 172a) is spaced along a portion of the width of the fastener tab (160, 160a); and
- where the fastener portions (172, 172a) of each closure member (136, 136a) are bonded to or formed on a body facing side of the fastener tab (160, 160a), and an outer surface of the fastener tab (160, 160a) of the first closure member (136, 136a) is configured to be engaged by the fastener portions (172, 172a) of the second closure member.

14. The method of claim 13, **characterized in that** releasably coupling the second ends (156a) of the closure members (136, 136a) comprises releasably coupling the fastener portions (172, 172a) of the first closure member (136, 136a) to the front waist portion (108) of the chassis (104), and releasably coupling at least one of the fastener portions (172, 172a) of the second closure member (136, 136a) to both the outer side of the fastener tab (160, 160a) of the first closure member (136, 136a) and the front waist portion (108).

15. The method of claim 12, **characterized in that** the elastic panels of each closure member (136, 136a) are bonded directly to a body facing side of the elastic panel of that closure member (136, 136a), and **in that**
- coupling the second ends (156a) of the closure members (136, 136a) comprises releasably coupling the fasteners of each closure member (136, 136a) to the front waist portion (108) such that the second ends (156a) of the closure members (136, 136a) do not overlap; or
- an outer side of the elastic panel of the first closure members (136, 136a) is configured to be engaged by the fastener portions (172, 172a) of the second closure member, and coupling the second ends (156a) of the first and second closure members (136, 136a) comprises releasably coupling the fastener portions (172, 172a) of the first closure member to the front waist portion (108) of the chassis (104), and releasably coupling at least one of the fastener portions (172, 172a) of the second closure member (136, 136a) to the outer side of the elastic panel of the first closure member (136, 136a).

## Patentansprüche

1. Ein wegwerfbarer absorbierender Artikel (100, 100a), aufweisend eine Hülle (104) mit
- gegenüberliegenden vorderen und hinteren Taillenabschnitten (108, 112),
- einen sich in Längsrichtung zwischen den vorderen und hinteren Taillenabschnitten (108, 112) erstreckenden Schrittabschnitt (116),
- eine dem Körper zugewandte Oberfläche (132), die so konfiguriert ist, dass sie während der Verwendung des Artikels (100, 100a) einem Träger zugewandt ist,
- eine äußere Oberfläche (128), die so konfiguriert ist, dass sie während der Verwendung des Artikels (100, 100a) von einem Träger abgewandt ist,
- ein erstes Verschlusselement (136) und ein zweites Verschlusselement (136a), wobei jedes Verschlusselement ein erstes Ende (152a), ein zweites Ende (156a) und eine sich zwischen den ersten und zweiten Enden erstreckende Breite hat, wobei jedes der Verschlusselemente (136, 136a) einen elastischen Bereich und eine Vielzahl von Befestigungsabschnitten (172, 172a) aufweist, die mit einem Abschnitt der Breite des Verschlusselements (136, 136a) verbunden sind und entlang diesem beabstandet sind,
**dadurch gekennzeichnet, dass**
- das erste Ende (152a) jedes Verschlusselements (136, 136a) mit dem hinteren Taillenabschnitt (112) der Hülle (104) verbunden ist, und das zweite Ende (156a) jedes Verschlusselements (136, 136a) dazu konfiguriert ist, den vorderen Taillenabschnitt (108) zu überlappen und lösbar mit diesem gekoppelt zu werden, um eine geschlossene Konfiguration zu definieren, in welcher
- die vorderen und hinteren Taillenabschnitte (108, 112) mit den Verschlusselementen (136, 136a) zusammenwirken, um eine Taillenöffnung zu umgeben und zu definieren
- eine linke Seite der Hülle (104) eine erste Beinöffnung (216) definiert, und
- eine rechte Seite der Hülle (104) eine zweite Beinöffnung (220) definiert,
die Vielzahl von Befestigungsabschnitten (172, 172a) des ersten Verschlusselements (136, 136a) konfiguriert sind, um den vorderen Taillenabschnitt (108) zu überlappen, wobei die Befestigungsabschnitte (172, 172a) des ersten Verschlusselements (136, 136a) lösbar mit dem vorderen Taillenabschnitt (108) verbunden sind (engaged), und
die Vielzahl von Befestigungsabschnitten (172, 172a) des zweiten Verschlusselements (136, 136a) konfiguriert sind, um das erste Verschlusselement (136, 136a) zu überlappen, wobei die Befestigungsabschnitte (172, 172a) des zweiten Verschlusselements (136, 136a) lösbar mit der Außenfläche der Befestigungsabschnitte (172, 172a) des ersten Verschlusselements (136, 136a) verbunden sind.

2. Der Artikel nach Anspruch 1, **dadurch gekennzeichnet, dass** der elastische Bereich jedes Verschlusselements (136, 136a) ein erstes Ende hat, welches das erste Ende (152a) des Verschlusselements (136, 136a) definiert, ein zweites Ende, und eine sich zwischen den ersten und zweiten Enden erstreckende Breite, und jedes Verschlusselement (136, 136a) weiterhin Folgendes aufweist:
- eine Befestigungslasche (160, 160a) mit einem ersten Ende (164), welches mit dem elastischen Bereich verbunden ist, und einem zweiten Ende (168), welches ein zweites Ende (156a) des Verschlusselements definiert, so dass sich die Breite des Verschlusselements (136, 136a) vom ersten Ende des elastischen Bereich zum zweiten Ende der Befestigungslasche (160, 160a) erstreckt;
- wobei die Vielzahl von Befestigungsabschnitten (172, 172a) entlang eines Abschnitts der Breite der Befestigungslasche (160, 160a) beabstandet ist.

3. Der Artikel nach Anspruch 2, **dadurch gekennzeichnet, dass** die Befestigungsabschnitte (172, 172a) jedes Verschlusselements (136, 136a) an einer dem Körper zugewandten Seite der Befestigungslasche (160, 160a) des Verschlusselements (136, 136a) verbunden oder ausgebildet sind, und dass eine Außenfläche der Befestigungslasche (160, 160a) des ersten Verschlusselements (136,136a) konfiguriert ist, um mit den Befestigungsabschnitten (172, 172a) des zweiten Verschlusselements (136, 136a) verbunden zu werden.

4. Der Artikel nach Anspruch 3, **dadurch gekennzeichnet, dass** die Befestigungslasche (160, 160a) des ersten Verschlusselements (136, 136a) so konfiguriert ist, dass sie den vorderen Taillenabschnitt (108) überlappt, wobei die Befestigungsabschnitte (172, 172a) des ersten Verschlusselements lösbar mit einer Auflagezone (176, 176a) des vorderen Taillenabschnitts (108) verbunden, und die Befestigungslasche (160, 160a) des zweiten Verschlusselements (136, 136a) konfiguriert ist, um das erste Verschlusselement zu überlappen, wobei die Befestigungsabschnitte (172, 172a) des zweiten Verschlusselements (136, 136a) lösbar mit sowohl der Auflagezone (176, 176a) als auch der Außenfläche der Befestigungslasche (160, 160a) des ersten Verschlusselements (136, 136a) verbunden sind.

5. Der Artikel nach Anspruch 1, **dadurch gekennzeichnet, dass** die elastischen Bereiche jedes Verschlusselements (136, 136a)
- direkt mit einer dem Körper zugewandten Seite des elastischen Bereichs dieses Verschlusselements verbunden sind; oder
- direkt mit einer dem Körper zugewandten Seite des elastischen Bereichs dieses Verschlusselements (136, 136a) verbunden sind und dass eine Außenseite des elastischen Bereichs eines ersten der Verschlusselemente (136, 136a) konfiguriert ist, um mit den Befestigungsabschnitten (172, 172a) des zweiten Verschlusselements (136, 136a) verbunden zu werden; oder
- ein erstes Ende haben, welches das erste Ende (152a) des Verschlusselements (136, 136a) definiert, ein zweites Ende, und eine sich zwischen den ersten und zweiten Enden erstreckende Breite; die Hülle (104) hat eine Produkthöhe, die sich von einem Mittelpunkt des Schrittabschnitts zu einer äußersten Kante des hinteren Taillenabschnitts (112) erstreckt; und eine Länge des ersten Endes des elastischen Bereichs jedes Verschlusselements (136, 136a), parallel zur Produkthöhe gemessen, größer oder gleich 45% der Produkthöhe ist.

6. Der Artikel nach Anspruch 1, **dadurch gekennzeichnet, dass** eine Auflagezone (176, 176a, 176b) der Außenfläche auf dem vorderen Taillenabschnitt (108) der Hülle (104) konfiguriert ist, um mit den Befestigungsabschnitten (172, 172a) der Verschlusselemente (136, 136a) lösbar verbunden zu werden, wobei die Vielzahl von Befestigungsabschnitten (172, 172a, 172b) des zweiten Verschlusselements (136, 136a) ein erstes Befestigungselement und ein zweites Befestigungselement enthält, und das zweite Verschlusselement (136, 136a) so konfiguriert ist, dass es über den vorderen Taillenabschnitt (108) gefaltet wird, so dass das erste Befestigungselement lösbar mit der Außenfläche des ersten Verschlusselements (136, 136a) verbunden ist und das zweite Befestigungselement lösbar mit der Auflagezone (176, 176a, 176b) verbunden ist.

7. Der Artikel nach Anspruch 6, **dadurch gekennzeichnet, dass** die Auflagezone (176, 176a, 176b) der Außenfläche durch ein Verankerungselement definiert ist, das mit einer rückwärtigen Lage der Hülle (104) verbunden ist.

8. Der Artikel nach Anspruch 7, **dadurch gekennzeichnet, dass** das Verankerungselement ein Schlaufenmaterial aufweist, und die Befestigungsabschnitte (172, 172a) der Verschlusselemente (136, 136a) ein Hakenmaterial aufweisen, das so konfiguriert ist, dass es lösbar in das Schlaufenmaterial eingreift.

9. Der Artikel nach einem der Ansprüche 1 - 8, **dadurch gekennzeichnet, dass** die Befestigungsabschnitte (172, 172a) der Verschlusselemente (136, 136a) ein Hakenmaterial aufweisen, und wobei jeder Befestigungsabschnitt (172, 172a) der Vielzahl von Befestigungsabschnitten (172, 172a) des zweiten Verschlusselements (136, 136a) durch einen ersten Abstand (256) getrennt ist, welcher mindestens 50% einer Breite von mindestens einem der entsprechenden Befestigungsabschnitte (172, 172a) beträgt.

10. Der Artikel nach einem der Ansprüche 1 - 9, **dadurch gekennzeichnet, dass**
- der vordere Taillenabschnitt (108), der hintere Taillenabschnitt (112)und der Schrittabschnitt durch entsprechende Bereiche eines gemeinsamen Hülle (104)-Elements definiert sind; oder
- die Hülle (104) eine obere Lage aufweist, wobei die rückwärtige Lage mit der oberen Lage verbunden ist, und ein absorbierender Kern zwischen der oberen Lage und der rückwärtigen Lage angeordnet ist.

11. Eine Anordnung, aufweisend:
- eine Vielzahl von wegwerfbaren absorbierenden Artikeln nach einem der Ansprüche 1 bis 10 und
- eine Packung; und
**dadurch gekennzeichnet, dass** die eine Vielzahl von wegwerfbaren absorbierenden Artikeln in der Packung angeordnet sind, wobei sich jeder der Artikel in seiner geschlossenen Konfiguration befindet, in welcher sich die zweiten Enden (156a) seiner Verschlusselemente (136, 136a) überlappen und lösbar mit seinem vorderen Taillenabschnitt (108) verbunden sind.

12. Ein Verfahren zum Verpacken eines wegwerfbaren absorbierenden Artikels mit einer Hülle (104) mit gegenüberliegenden vorderen und hinteren Taillenabschnitten (108, 112), einem sich in Längsrichtung zwischen den vorderen und hinteren Taillenabschnitten (108, 112) erstreckenden Schrittabschnitt, und einer dem Körper zugewandten Oberfläche, die so konfiguriert ist, dass sie während der Verwendung des Artikels einem Träger zugewandt ist, **dadurch gekennzeichnet, dass** das Verfahren aufweist:
- Bereitstellen eines wegwerfbaren absorbierenden Artikels nach Anspruch 1;
- lösbares Koppeln der zweiten Enden (156a) der Verschlusselemente (136, 136a) des Artikels mit dem vorderen Taillenabschnitt (108) des Artikels, so dass die zweiten Enden (156a) der Verschlusselemente (136, 136a) den vorderen Taillenabschnitt (108) in der geschlossenen Konfiguration überlappen; und Anordnen des Artikels in der geschlossenen Konfiguration in einer Verpackung zum Vertrieb.

13. Das Verfahren nach Anspruch 12, **dadurch gekennzeichnet, dass** der elastische Bereich jedes Verschlusselements (136, 136a) ein erstes Ende (152a) hat, welches das erste Ende (152a) des Verschlusselements (136, 136a) definiert, ein zweites Ende, und eine sich zwischen den ersten und zweiten Enden erstreckende Breite, und jedes Verschlusselement (136, 136a) weiterhin aufweist:
- eine Befestigungslasche (160, 160a) mit einem ersten Ende, welches mit dem elastischen Bereich verbunden ist, und einem zweiten Ende, welches ein zweites Ende (156a) des Verschlusselements (136, 136a) definiert, so dass sich die Breite des Verschlusselements (136, 136a) vom ersten Ende des elastischen Bereichs zum zweiten Ende der Befestigungslasche (160, 160a) erstreckt;
- wobei die Vielzahl von Befestigungsabschnitten (172, 172a) entlang eines Abschnitts der Breite der Befestigungslasche (160, 160a) beabstandet ist; und
- wobei die Befestigungsabschnitte (172, 172a) jedes Verschlusselements (136, 136a) an einer dem Körper zugewandten Seite der Befestigungslasche (160, 160a) verbunden oder an dieser ausgebildet sind, und eine Außenfläche der Befestigungslasche (160, 160a) des ersten Verschlusselements (136, 136a) konfiguriert ist, um mit den Befestigungsabschnitten (172, 172a) des zweiten Verschlusselements verbunden zu werden.

14. Das Verfahren nach Anspruch 13, **dadurch gekennzeichnet, dass** lösbares Koppeln der zweiten Enden (156a) der Verschlusselemente (136, 136a) ein lösbares Koppeln der Befestigungsabschnitte (172, 172a) des ersten Verschlusselements (136, 136a) mit dem vorderen Taillenabschnitt (108) der Hülle (104), und lösbares Koppeln von mindestens einem der Befestigungsabschnitte (172, 172a) des zweiten Verschlusselements (136, 136a) an sowohl die Außenseite der Befestigungslasche (160, 160a) des ersten Verschlusselements (136, 136a) als auch den vorderen Taillenabschnitt (108) aufweist.

15. Das Verfahren nach Anspruch 12, **dadurch gekennzeichnet, dass** die elastischen Bereiche jedes Verschlusselements (136, 136a) direkt mit einer dem Körper zugewandten Seite des elastischen Bereichs dieses Verschlusselements (136, 136a) verbunden werden, und dass
- Koppeln der zweiten Enden (156a) der Verschlusselemente (136, 136a) ein lösbares Koppeln der Befestigungselemente jedes Verschlusselements (136, 136a) mit dem vorderen Taillenabschnitt (108) aufweist, so dass sich die zweiten Enden (156a) der Verschlusselemente (136, 136a) nicht überlappen; oder
- eine Außenseite des elastischen Bereichs des ersten Verschlusselements (136, 136a) konfiguriert ist, um mit den Befestigungsabschnitten (172, 172a) des zweiten Verschlusselements verbunden zu werden, und Koppeln der zweiten Enden (156a) der ersten und zweiten Verschlusselemente (136, 136a) ein lösbares Koppeln der Befestigungsabschnitte (172, 172a) des ersten Verschlusselements mit dem vorderen Taillenabschnitt (108) der Hülle (104), und ein lösbares Koppeln von mindestens einem der Befestigungsabschnitte (172, 172a) des zweiten Verschlusselements (136, 136a) mit der Außenseite des elastischen Bereichs des ersten Verschlusselements (136, 136a) aufweist.

## Revendications

1. Article absorbant jetable (100, 100a) comprenant un châssis (104) comportant :
- des portions de taille avant et arrière (108, 112) opposées,
- une portion d'entrejambes (116) s'étendant longitudinalement entre les portions de taille avant et arrière (108, 112),
- une surface faisant face au corps (132) configurée pour faire face à un porteur pendant une utilisation de l'article (100, 100a),
- une surface externe (128) configurée pour tourner le dos à un porteur pendant une utilisation de l'article (100, 100a),
- un premier organe de fermeture (136) et un second organe de fermeture (136a), chaque organe de fermeture ayant une première extrémité (152a), une seconde extrémité (156a), et une largeur s'étendant entre les première et seconde extrémités, chacun des organes de fermeture (136, 136a) comprenant un panneau élastique et une pluralité de portions de fixation (172, 172a) couplées à une portion de la largeur de l'organe de fermeture (136, 136a) et espacées de celle-ci,
**caractérisé en ce que** :
- la première extrémité (152a) de chaque organe de fermeture (136, 136a) est liée à la portion de taille arrière (112) du châssis (104), et la seconde extrémité (156a) de chaque organe de fermeture (136, 136a) est configurée pour chevaucher la portion de taille avant (108) et être couplée de façon libérable avec celle-ci pour définir une configuration fermée dans laquelle :
- les portions de taille avant et arrière (108, 112) coopèrent avec les organes de fermeture (136, 136a) pour encercler et définir une ouverture de taille,
- un côté gauche du châssis (104) définit une première ouverture de jambe (216), et
- un côté droit du châssis (104) définit une seconde ouverture de jambe (220),
la pluralité de portions de fixation (172, 172a) du premier organe de fermeture (136, 136a) sont configurées pour chevaucher la portion de taille avant (108), les portions de fixation (172, 172a) du premier organe de fermeture (136, 136a) étant engagées de façon libérable avec la portion de taille avant (108), et
la pluralité de portions de fixation (172, 172a) du second organe de fermeture (136, 136a) sont configurées pour chevaucher le premier organe de fermeture (136, 136a), les portions de fixation (172, 172a) du second organe de fermeture (136, 136a) étant engagées de façon libérable avec la surface externe des portions de fixation (172, 172a) du premier organe de fermeture (136, 136a).

2. Article selon la revendication 1, **caractérisé en ce que** le panneau élastique de chaque organe de fermeture (136, 136a) a une première extrémité définissant la première extrémité (152a) de l'organe de fermeture (136, 136a), une seconde extrémité, et une largeur s'étendant entre les première et seconde extrémités, et chaque organe de fermeture (136, 136a) comprend en outre :
- une languette de fixation (160, 160a) ayant une première extrémité (164) liée au panneau élastique, et une seconde extrémité (168) définissant une seconde extrémité (156a) de l'organe de fermeture de sorte que la largeur de l'organe de fermeture (136, 136a) s'étende de la première extrémité du panneau élastique à la seconde extrémité de la languette de fixation (160, 160a) ;
- où la pluralité de portions de fixation (172, 172a) sont espacées le long d'une portion de la largeur de la languette de fixation (160, 160a).

3. Article selon la revendication 2, **caractérisé en ce que** les portions de fixation (172, 172a) de chaque organe de fermeture (136, 136a) sont liées à un côté faisant face au corps, ou formées sur celui-ci, de la languette de fixation (160, 160a) de l'organe de fermeture (136, 136a), et une surface externe de la languette de fixation (160, 160a) du premier organe de fermeture (136, 136a) est configurée pour être engagée par les portions de fixation (172, 172a) du second organe de fermeture (136, 136a).

4. Article selon la revendication 3, **caractérisé en ce que** la languette de fixation (160, 160a) du premier organe de fermeture (136, 136a) est configurée pour chevaucher la portion de taille avant (108), les portions de fixation (172, 172a) du premier organe de fermeture étant engagées de façon libérable avec une zone d'accueil (176, 176a) de la portion de taille avant (108), et la languette de fixation (160, 160a) du second organe de fermeture (136, 136a) est configurée pour chevaucher le premier organe de fermeture, les portions de fixation (172, 172a) du second organe de fermeture (136, 136a) étant engagées de façon libérable à la fois avec la zone d'accueil (176, 176a) et la surface externe de la languette de fixation (160, 160a) du premier organe de fermeture (136, 136a).

5. Article selon la revendication 1, **caractérisé en ce que** les panneaux élastiques de chaque organe de fermeture (136, 136a)
- sont liés directement à un côté faisant face au corps du panneau élastique de cet organe de fermeture ; ou
- sont liés directement à un côté faisant face au corps du panneau élastique de cet organe de fermeture (136, 136a) et **en ce qu'**un côté externe du panneau élastique d'un premier des organes de fermeture (136, 136a) est configuré pour être engagé par les portions de fixation (172, 172a) du second organe de fermeture (136, 136a) ; ou
- comportent une première extrémité définissant la première extrémité (152a) de l'organe de fermeture (136, 136a), une seconde extrémité, et une largeur s'étendant entre les première et seconde extrémités ; le châssis (104) a une hauteur de produit s'étendant d'un point médian de la portion d'entrejambes à un bord le plus externe de la portion de taille arrière (112) ; et une longueur de la première extrémité du panneau élastique de chaque organe de fermeture (136, 136a), mesurée parallèlement à la hauteur de produit, est supérieure ou égale à 45 % de la hauteur de produit.

6. Article selon la revendication 1, **caractérisé en ce qu'**une portion d'accueil (176, 176a, 176b) de la surface externe sur la portion de taille avant (108) du châssis (104) est configurée pour être engagée de façon libérable par les portions de fixation (172, 172a) des organes de fermeture (136, 136a), la pluralité de portions de fixation (172, 172a, 172b) du second organe de fermeture (136, 136a) comportent une première fixation et une seconde fixation, et le second organe de fermeture (136, 136a) est configuré pour être replié sur la portion de taille avant (108) de sorte que la première fixation soit engagée de façon libérable avec la surface externe du premier organe de fermeture (136, 136a) et la seconde fixation soit engagée de façon libérable avec la portion d'accueil (176, 176a, 176b).

7. Article selon la revendication 6, **caractérisé en ce que** la portion d'accueil (176, 176a, 176b) de la surface externe est définie par un organe d'ancrage lié à une feuille arrière du châssis (104).

8. Article selon la revendication 7, **caractérisé en ce que** l'organe d'ancrage comprend un matériau à boucles, et les portions de fixation (172, 172a) des organes de fermeture (136, 136a) comprennent un matériau à crochets configuré pour s'engager de façon libérable avec le matériau à boucles.

9. Article selon l'une quelconque des revendications 1 à 8, **caractérisé en ce que** les portions de fixation (172, 172a) des organes de fermeture (136, 136a) comprennent un matériau à crochets, et dans lequel chaque portion de fixation (172, 172a) de la pluralité de portions de fixation (172, 172a) du second organe de fermeture (136, 136a) est séparée d'une première distance (256) qui est d'au moins 50 % d'une largeur d'au moins l'une des portions de fixation (172, 172a) respectives.

10. Article selon l'une quelconque des revendications 1 à 9, **caractérisé en ce que**
- la portion de taille avant (108), la portion de taille arrière (112), et la portion d'entrejambes sont définies par des régions correspondantes d'un organe de châssis (104) commun ; ou
- le châssis (104) comprend une feuille supérieure, une feuille arrière liée à la feuille supérieure, et une âme absorbante disposée entre la feuille supérieure et la feuille arrière.

11. Ensemble comprenant :
- une pluralité d'articles absorbants jetables de l'une quelconque des revendications 1 à 10 et
- un emballage ; et
**caractérisé en ce que** la pluralité d'articles absorbants jetables sont disposés dans l'emballage, chacun des articles étant dans sa configuration fermée dans laquelle les secondes extrémité (156a) de ses organes de fermeture (136, 136a) se chevauchent et sont couplées de façon libérable avec sa portion de taille avant (108).

12. Procédé d'emballage d'un article absorbant jetable comportant un châssis (104) avec des portions de taille avant et arrière (108, 112) opposées, une portion d'entrejambes s'étendant longitudinalement entre les portions de taille avant et arrière (108, 112), et une surface faisant face au corps configurée pour faire face à un porteur pendant l'utilisation de l'article, **caractérisé en ce que** le procédé comprend :
- la fourniture d'un article absorbant jetable de la revendication 1 ;
- le couplage libérable des secondes extrémité (156a) des organes de fermeture (136, 136a) de l'article avec la portion de taille avant (108) de l'article de sorte que les secondes extrémités (156a) des organes de fermeture (136, 136a) chevauchent la portion de taille avant (108) dans la configuration fermée ; et la disposition de l'article dans la configuration fermée dans un emballage en vue d'une distribution.

13. Procédé selon la revendication 12, **caractérisé en ce que** le panneau élastique de chaque organe de fermeture (136, 136a) a une première extrémité (152a) définissant la première extrémité (152a) de l'organe de fermeture (136, 136a), une seconde extrémité, et une largeur s'étendant entre les première et seconde extrémités, et chaque organe de fermeture (136, 136a) comprend en outre :
- une languette de fixation (160, 160a) ayant une première extrémité liée au panneau élastique, et une seconde extrémité définissant une seconde extrémité (156a) de l'organe de fermeture (136, 136a) de sorte que la largeur de l'organe de fermeture (136, 136a) s'étende de la première extrémité du panneau élastique à la seconde extrémité de la languette de fixation (160, 160a) ;
- où la pluralité de portions de fixation (172, 172a) sont espacées le long d'une portion de la largeur de la languette de fixation (160, 160a) ; et
- où les portions de fixation (172, 172a) de chaque organe de fermeture (136, 136a) sont liées à un côté faisant face au corps, ou formées sur celui-ci, de la languette de fixation (160, 160a), et une surface externe de la languette de fixation (160, 160a) du premier organe de fermeture (136, 136a) est configurée pour être engagée par les portions de fixation (172, 172a) du second organe de fermeture.

14. Procédé selon la revendication 13, **caractérisé en ce que** le couplage libérable des secondes extrémités (156a) des organes de fermeture (136, 136a) comprend le couplage libérable des portions de fixation (172, 172a) du premier organe de fermeture (136, 136a) avec la portion de taille avant (108) du châssis (104), et le couplage libérable d'au moins l'une des portions de fixation (172, 172a) du second organe de fermeture (136, 136a) à la fois avec le côté externe de la languette de fixation (160, 160a) du premier organe de fermeture (136, 136a) et avec la portion de taille avant (108).

15. Procédé selon la revendication 12, **caractérisé en ce que** les panneaux élastiques de chaque organe de fermeture (136, 136a) sont liés directement à un côté faisant face au corps du panneau élastique de cet organe de fermeture (136, 136a), et **en ce que**
- le couplage des secondes extrémités (156a) des organes de fermeture (136, 136a) comprend le couplage libérable des fixations de chaque organe de fermeture (136, 136a) avec la portion de taille avant (108) de sorte que les secondes extrémités (156a) des organes de fermeture (136, 136a) ne se chevauchent pas ; ou
- un côté externe du panneau élastique du premier organe de fermeture (136, 136a) est configuré pour être engagé par les portions de fixation (172, 172a) du second organe de fermeture, et le couplage des secondes extrémités (156a) des premier et second organes de fermeture (136, 136a) comprend le couplage libérable des portions de fixation (172, 172a) du premier organe de fermeture avec la portion de taille avant (108) du châssis (104), et le couplage libérable d'au moins l'une des portions de fixation (172, 172a) du second organe de fermeture (136, 136a) avec le côté externe du panneau élastique du premier organe de fermeture (136, 136a).
